# EUROPEAN PATENT SPECIFICATION

(11) **EP 0 504 919 B1**
(45) Date of publication and mention of the grant of the patent: **04.09.1996**
(21) Application number: 92104879.9
(22) Date of filing: 20.03.1992
(51) Int. Cl.: C12N 15/29, C12N 1/21, C07K 14/415

(54) **Mirabilis jalapa antiviral protein**
Antiviren-Protein aus Mirabilis jalapa
Protéine antivirale extraite de Mirabilis jalapa

(30) Priority: 22.03.1991 JP 83456/91
(43) Date of publication of application: 23.09.1992
(73) Proprietor: Japan Tobacco Inc., Shinagawa-ku, Tokyo 140 (JP)
(72) Inventor: Habuka, Noriyuki, c/o Japan Tobacco Inc., Yokohama-shi (JP); Miyano, Masashi, c/o Japan Tobacco Inc., Yokohama-shi (JP); Matsumoto, Takashi, c/o Japan Tobacco Inc., Yokohama-shi (JP); Noma, Masana, c/o Japan Tobacco Inc., Yokohama-shi (JP)
(74) Representative: Reinhard - Skuhra - Weise & Partner

(56) References cited:
- JOURNAL OF BIOLOGICAL CHEMISTRY (MICROFILMS), vol. 265, no. 19, 05 July 1990, Baltimore, MD (US); N. HABUKA et al., pp. 10988-10992
- JOURNAL OF BIOLOGICAL CHEMISTRY (MICROFILMS), vol. 264, no. 12, 25 April 1989, Baltimore, MD (US); N. HABUKA et al., pp. 6629-6637
- S. OLSNES et al., "Molecular Aspects of Cellular Regulation", Cohen & Van Heyningen (eds.), vol 2, chapter 3, 1982, Elsevier Biomedical Press, Amsterdam (NL); pp. 53-105
- TIBS TRENDS IN BIOCHEMICAL SCIENCES, vol. 15, January 1990, Cambridge (GB); C.N. PACE, pp. 14-17
- JOURNAL OF BIOLOGICAL CHEMISTRY (MICROFILMS), vol. 266, no. 35, 15 December 1991, Baltimore, MD (US); N. HABUKA et al., pp. 23558-23560

## Description

The present invention relates to a Mirabilis Antiviral Protein (to be referred to as MAP hereinafter) variant and, more particularly, to an MAP variant whose inhibition activity in vitro protein synthesis is improved as compared with natural MAP.

We, the present inventors, previously separated a novel basic protein from Mirabilis jalapa and found that this protein showed an antiviral activity against a plurality of plant viruses. This protein was called MAP and was claimed in Published Examined Japanese Patent Application No. 63-61317. To specify all amino acid sequences, to completely synthesize a gene based on the specified amino acid sequences, and to construct a system in E.coli, in which MAP is produced and secreted to a medium, using this complete or full synthetic MAP gene were claimed in Published Unexamined Japanese Patent Application No. 2-186988 and Japanese Patent Application No. 1-210767.

The MAP is a kind of ribosome inactivating protein (RIP) widely existing in plants and microorganisms and exhibits an RNA N-glycosidase activity with high specificity to a ribosomal RNA as a substrate. The ribosome is inactivated by this activity to inhibit protein synthesis, as is well known. The protein synthesis inhibition activity is highly toxic in a cell. In recent years, this toxicity is utilized to develop immunotoxins having high selectivity, for example, by linking a ricin A chain as a kind of RIP derived from Ricinus communis to various antibodies. These immunotoxins are utilized as an example in missile therapy.

This protein toxin has high antigenicity. There is a possibility that an antibody against the toxin is produced in a living body. Thus, a long-term dose may adversely affect the living body. Therefore, candidates having various properties as toxic proteins are required.

Under these circumstances, the MAP is one of the most promising candidates as a toxin for an immunotoxin. The protein synthesis inhibition activity for a rabbit reticulocyte system is, however, figured out to be only about 1/30 of the ricin A chain.

The protein engineering which involves with gene manipulation techniques has been remarkably developed in recent years. In various applications, amino acid sequences of natural proteins are converted to produce proteins whose inherent activities are changed. It is found in these applications that a disulfide bond (S-S bond) in a protein molecule is closely associated with flexibility of a protein molecule structure, and that the activity is greatly changed depending on the presence/absence of the S-S bond (Matsumura et. al., Nature 342, pp. 291 - 293 (1989); Matsumura et. al., Pro. Natl. Acad. Sci. USA 87, pp. 6562 - 6566 (1989); Kozo Hamaguchi, Biochemistry 1, pp. 1 - 13 (1991); and Pace, Biotrend 2-4, pp. 105 - 110 (1990)).

When the above background is taken into consideration, a production of protein having a high value and, more particularly, having a high protein synthesis inhibition activity is expected by utilizing MAP genes.

It is an object of the present invention to provide a protein having a high value and, more particularly, a protein having a higher protein synthesis inhibition activity on the basis of MAP.

We have made extensive studies to achieve the above object and have found that an MAP variant having a higher protein synthesis inhibition activity than that of MAP by changing two cysteine residues associated with an S-S bond of the MAP into serine residues. That is, the antiviral protein according to the present invention is an MAP variant having an amino acid sequence represented as SEQ ID NO:1 in the Sequence Listing to be described later.

This MAP variant having no cysteine bond (to be referred to as MAP-H hereinafter) is produced such that a codon for coding the cysteine is converted into a codon for coding the serine in the MAP gene, that the resultant gene is inserted into an MAP secretion vector, and that this vector is introduced into a host such as E.coli.

The MAP gene is a gene for coding MAP. An amino acid and a codon for specifying this amino acid are not generally set in a one-to-one correspondence. One to six types of codons for specifying one amino acid are generally present. A large number of types of MAP genes are present, and then a large number of types of recombinant genes in which codons for coding cysteines are substituted with codons for coding serines are present accordingly. When this recombinant gene is to be introduced into a host cell to produce an antiviral protein of the present invention, all the recombinant genes can be utilized. When a specific amino acid is taken into consideration, however, the frequency of use in several types of codons for coding the specific amino acid is unbalanced depending on each living species. Thus, when E.coli is a host, a gene having a base sequence represented as SEQ ID No:2 in the Sequence Listing is preferably used.

Production of an MAP gene based on an MAP amino acid sequence is described in Japanese Patent Application No. 63-93494 in detail. According to this method, a codon for coding cysteine is substituted with a codon for coding serine to obtain a total synthetic gene for coding the antiviral protein of the present invention. An E.coli transformant having a vector containing a total synthetic MAP gene produced by the above method has already been deposited in Fermentation Research Institute (FRI) as deposit No. 9913. A total synthetic MAP gene can be extracted from this transformant, and only the codon for coding cysteine is substituted with the codon for coding serine to obtain a gene for coding the antiviral protein of the present invention.

This invention can be more fully understood from the following detailed description when taken in conjunction with the accompanying drawings, in which:
Fig. 1 is a view showing a base sequence of a synthetic gene for coding MAP;
Fig. 2 is a view showing an OmpA signal sequence and a base sequence of a gene for coding the OmpA signal sequence together with part of an MAP amino acid sequence and a base sequence of a gene for coding the MAP;
Fig. 3 is a view showing a base sequence of a synthetic DNA linker;
Fig. 4 is a view schematically illustrating step A in an Example of the present invention;
Fig. 5 is a view schematically illustrating step B in the Example of the present invention;
Fig. 6 is a view schematically illustrating step C in the Example of the present invention;
Fig. 7 is a view schematically illustrating step E in an Example of the present invention;
Fig. 8 is a view schematically illustrating steps F and G in the Example of the present invention;
Fig. 9 is a view schematically illustrating steps H and I in the Example of the present invention;
Fig. 10 is a view schematically illustrating steps J and K in the Example of the present invention;
Fig. 11 is a view showing an amino acid sequence of block II inserted in an MAP variant and a base sequence of a DNA fragment synthesized for coding this amino acid sequence according to the Example of the present invention;
Fig. 12 is a view showing an amino acid sequence of block VII inserted in an MAP variant and a base sequence of a DNA fragment synthesized for coding this amino acid sequence according to the Example of the present invention;
Fig. 13 is a photograph showing a migration pattern of an MAP variant by SDS polyacrylamide gel electromigration; and
Fig. 14 is a graph showing protein synthesis inhibition activities of MAP and an MAP variant having no S-S bond with respect to a rabbit reticulocyte system.

A method of preparing an antiviral protein according to the present invention will be described in detail wherein a total MAP gene extracted from a deposited E.coli transformant is used.

### I) Preparation of A MAP Gene Fragment in which A Codon for coding Cystine is Substituted with A Codon for coding Serine

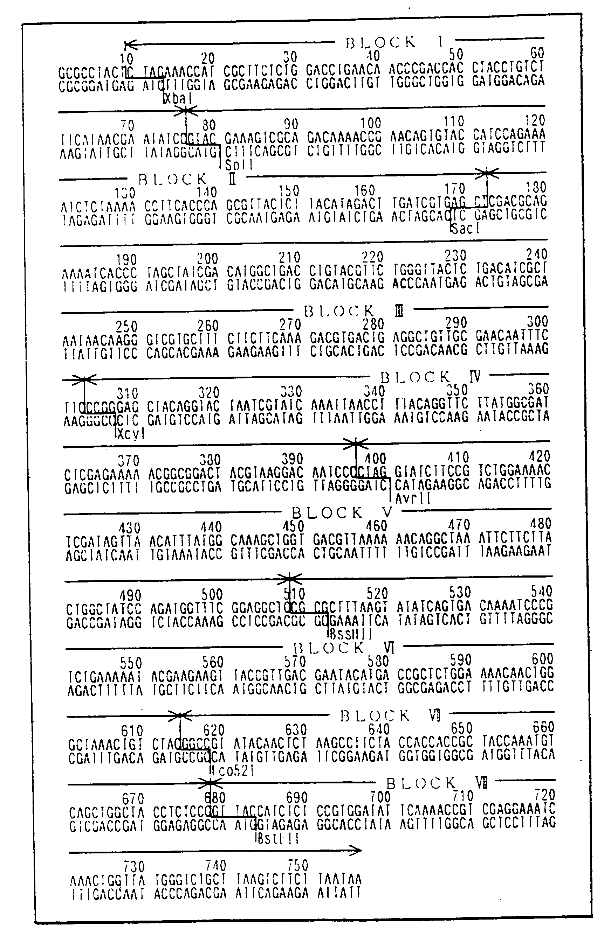

A total synthetic MAP gene (SEQ ID NO:5) contained in the deposited E.coli transformant is constructed by linking eight blocks I to VIII shown in Fig. 1. The blocks are linked at different restriction sites, respectively. Only a desired block can be obtained by using a specific restriction enzyme. Codons for coding two cysteine residues present in the MAP amino acid sequence are respectively present in blocks II and VII of this total synthetic MAP gene. In order to obtain a MAP gene for coding MAP variant of the present invention, DNA fragments of these portions are newly synthesized with substitution of a codon for coding cysteine by a codon for coding serine, and then the MAP gene is reconstructed using the new fragments in place of fragments of blocks II and VII in units of blocks.

For this purpose, a DNA fragment substituted with a target codon is synthesized and integrated in a plasmid. The plasmid containing this DNA fragment is cloned. At this time, it is desirable that the plasmid has same base sequence as original sequence except that a restriction enzyme site is additionally inserted or deleted, and that the plasmid is cloned by use of the site as a marker. Note that synthesis is performed by using a commercially available DNA synthesizer, and purification is performed using HPLC. Known methods are used in phenol treatment and ethanol precipitation necessary for treating DNA, DNA digestion or cleaving by a restriction enzyme, and the subsequent DNA collection, and a calcium method serving as an E.coli transformation method using the plasmid DNA, an alkaline-SDS method serving as a method of purifying DNA from an E.coli transformant, and a dideoxy method serving as a sequence determination for nucleic acid. In cloning, a presynthesized DNA is annealed such that terminals of the resultant double-stranded DNA having EcoRI and HindIII sites and the resultant double-stranded DNA are easily inserted into the plasmid, at which the same sites with the terminals of the resultant double-stranded DNA are present.

### II) Insertion of Cloned DNA Fragment into MAP Gene

Since the terminals of each block of the MAP gene form restriction enzyme sites, the each cloned DNA fragment is cleaved at corresponding sites, and the cleaved fragment is then inserted into a total synthetic MAP gene. Insertion of each fragment is confirmed by the presence/absence of a newly inserted restriction enzyme site or a deleted restriction enzyme site. The inserted each MAP gene is cleaved again by an appropriate restriction enzyme, and the cleaved portions are linked to each other to complete production of an MAP variant (i.e., MAP-H gene).

### III) Production of E.coli Expression Vector

A vector for introducing a foreign gene which is to be expressed in E.coli generally requires the following DNA sequence other than the gene to be introduced;
(a) a region for operating transcription (operator)
(b) a region for promoting initiation of gene transcription (promoter);
   Examples of promoters which are known to manifest in E.coli are N25 promoter and P_{L} promoter both of which are derived from coliphage, etc.. The P_{L} promoter is derived from E:coli lambda-phage and is known to be repressed by a control protein which is called cI. The cI includes temperature-sensitive variant, called cI₈₅₇, which represses P_{L} promoter at 30°C, like the cI, but loses its repressing ability at 42°C, resulting in P_{L} promoter turn on. Accordingly, if an expression vector introduced in E.coli includes both P_{L} promoter and cI₈₅₇ repressor gene, E.coli can be grown by culturing it at 30°C such that PL promoter is repressed. Also, P_{L} promoter can be turned on to initiate transcription of the gene by culturing E.coli at 42°C.
   The P_{L} promoter can be obtained by digesting lambda-phage gene or disclosed pPL-lambda plasmid with restriction enzyme BamHI and HpaI. The cI₈₅₇ repressor gene can be obtained by digesting gene of lambda-phage variant (cI₈₅₇, Sam 7) with restriction enzyme BglII and BanIII.
(c) a region for instructing termination of transcription (terminator)
   Examples of known terminators are tLI terminator derived from coliphage, rrnBT₁T₂ terminator derived from ribosome gene of E.coli, etc..
(d) a region for instructing initiation position of transcription after transcripting into mRNA (Shine-Dalgarno, SD sequence)
   A sequence which is common to construction gene of E.coli can be used as SD sequence.
(e) methionine codon which is linked to the SD sequence and is for initiation of translation (ATG).

An expression vector derived from plasmid DNA can be formed by deletion or insertion of a specific region from and in the plasmid DNA. The deletion and insertion can be performed by cleaving the plasmid at specific sites and combining the resultant fragments by means of an appropriate treatment. Specifically, appropriate utilization of synthetic DNA fragments enables restriction enzyme sites, SD sequence, gene for coding amino acid sequence of protein, etc. which are not included in the original plasmid DNA to introduce.

For example, by making the DNA fragment including XbaI and BanIII sites that are not included in plasmid pKK223-3 and its complementary chain to combine with a cleavage fragment (a large fragment) which is obtained by digesting the pKK223-3 with EcoRI and HindIII, the restriction enzyme sites of XbaI and BanIII can be introduced in the pKK223-3. Additionally, by utilizing this pKK223-3 which includes restriction enzyme sites of XbaI and BanIII and a total synthetic MAP gene also having XbaI and BanIII sites in its 5'- and 3'-terminals respectively, the total synthetic MAP gene can be introduced into the plasmid pKX223-3. Further, SD sequence and a codon for coding a methionine residue which is required for initiating transcription of gene can also be introduced into said plasmid at this time. Moreover, since restriction enzyme site of NdeI (CATATG) includes a methionine codon (ATG) in, a gene for coding other proteins can be introduced through this site.

Required DNA fragments can be synthesized by a DNA synthesizer. An expression vector is formed by combining DNA fragments obtained by the DNA synthesizer, those obtained by restriction enzyme cleavage, and such a DNA fragment as needed obtained by converting a cohesive end into a flush end utilizing T4 DNA polymerase, DNA polymerase Klenow fragment, etc.. Each fragment can be combined by T4 DNA ligase or a commercially available ligation kit including the DNA ligase.

### IV) Production of MAP Secretion Vector

As described above, the total synthetic MAP gene having, e.g., P_{L} promotor and cI₈₅₇ gene and extracted from the E.coli transformant (deposit No. 9913) deposited in FRI is integrated in an expression vector, and the expression vector is introduced into E.coli, thereby expressing the gene and hence mass-produce MAP in E.coli. In this case, the mass-produced MAP is stored in only E.coli. This MAP affects a growth of E.coli and, thus, the production of MAP is limited. The mass-produced protein must be secreted outside the E.coli.

OmpA is an outer membrane protein of E.coli and comprises a signal sequence (SEQ ID No:6) as shown in Fig. 2. A base sequence of a gene for coding this signal sequence is also shown in Fig. 2. This signal sequence functions to make OmpA to secrete from E.coli. Therefore, by linking this signal sequence to N-terminal of other protein, the protein can be transferred outside from inside of the E.coli. For example, by linking the signal sequence shown in Fig. 2 to N-terminal of MAP, MAP can be secreted from E.coli.

Further, a gene for coding the signal sequence has the first methionine codon included in a part of said NdeI sites, and thus a foreign gene can be introduced into an expression vector having the NdeI site which is downstream from a promoter.

Fig. 2 also shows a sequence of three amino acid of MAP N-terminal and a DNA sequence corresponding thereto.

### V) Insertion of MAP-H Gene into Secretion Vector

The MAP secretion vector obtained in step IV has a gene containing the OmpA signal sequence, and the MAP gene is inserted in the downstream thereof. The cleaving site of restriction enzyme XbaI is present in the N-terminal sequence of the MAP gene, and the BanIII site is present in the downstream of the C-terminal. Therefore, the MAP-H gene obtained in step II is inserted using these two sites.

### VI) Mass Production and Purification of MAP-H

If the MAP secretion vector used in step V has, e.g., P_{L} promotor and cI₈₅₇ coding for its control protein, expression of the MAP gene located in the downstream of the promotor is completely repressed at 30°C, and cI₈₅₇ is inactivated at 42°C to immediately express the MAP gene, thereby producing the MAP. A signal sequence of E.coli outer membrane protein OmpA is inserted in the upstream of the MAP gene, so that the produced MAP is immediately secreted into the medium. A transformant containing the plasmid into which the MAP-H gene is inserted is cultured at a low temperature. When the concentration of the transformant has an appropriate value, the MAP-H gene is expressed by using a known temperature shift. The MAP-H is produced from E.coli and is secreted into the medium. The resultant MAP-H is condensed as a precipitate by salting out with ammonium sulfate and is then dialyzed. The dialyzed MAP-H is purified using Carboxymethyl Sepharose and Blue Sepharose column chromatography.

The MAP or MAP-H as an MAP variant is quantitatively evaluated by an ELISA (Enzyme-Linked Immunosorbent Assay) using an MAP antiserum against MAP. In vitro protein synthesis, i.e., translation, the RNA of tobacco mosaic virus is added as mRNA in a protein synthesis system of a commercially available rabbit reticulocyte crude extract, and the evaluation is performed using the content of labeled amino acid (³⁵S-methionine) taken in the acid-insoluble polypeptide produced by the translation. At this time, an appropriate amount of MAP or MAP-H is added to the system to quantitatively evaluate an influence on this protein synthesis.

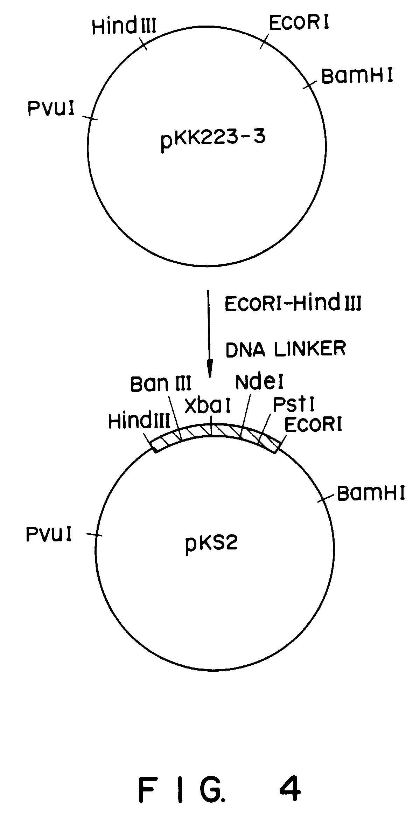

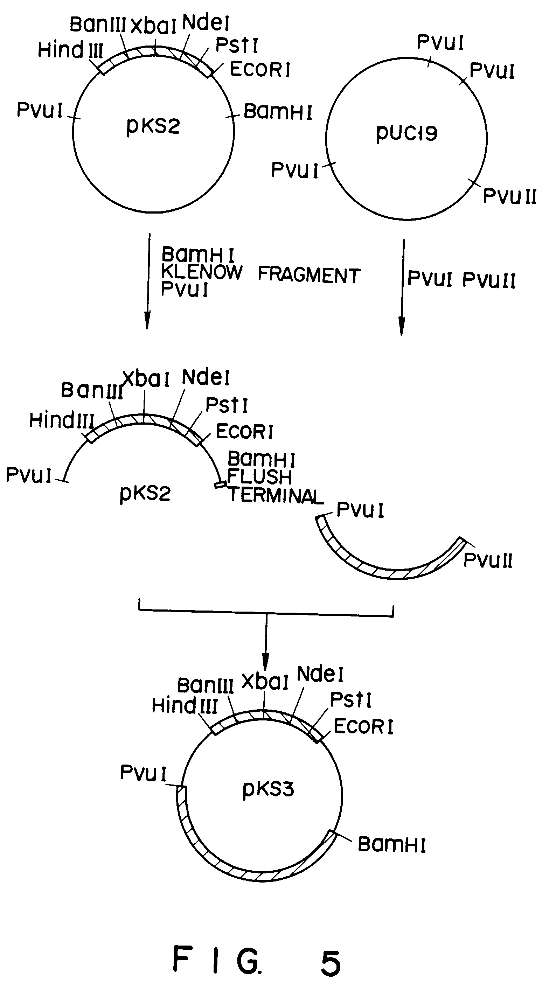

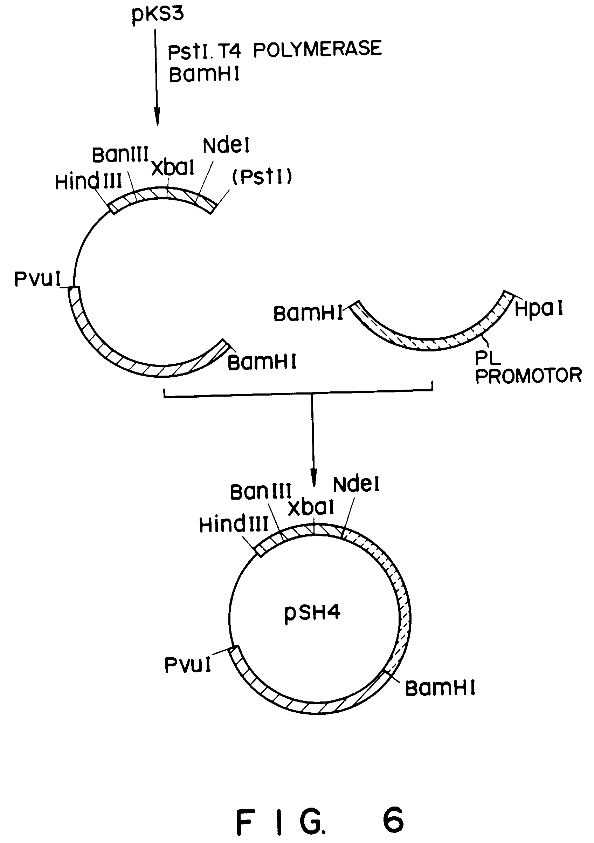

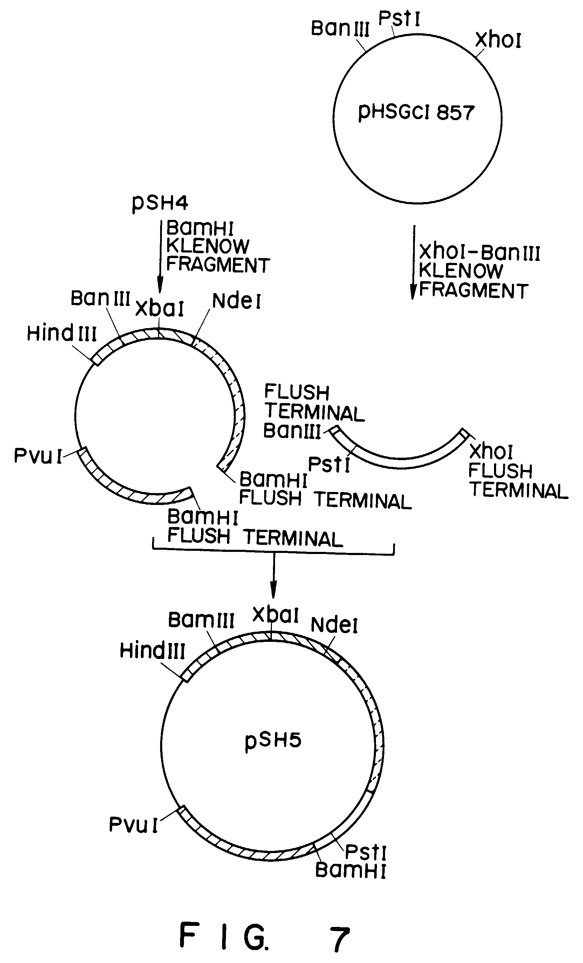

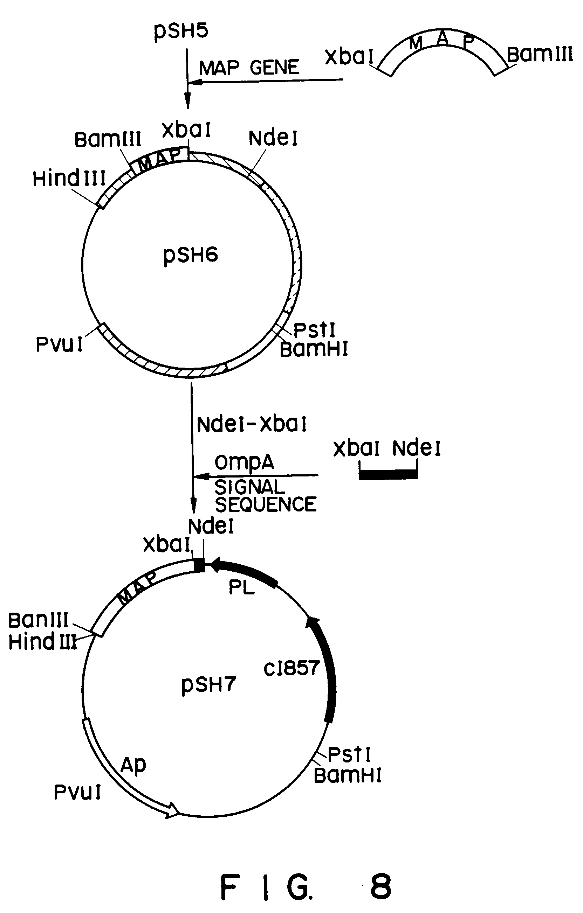

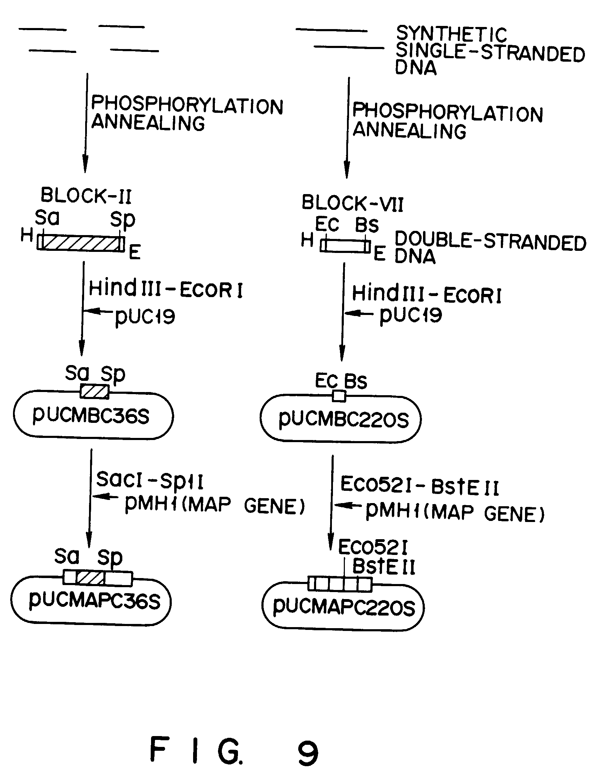

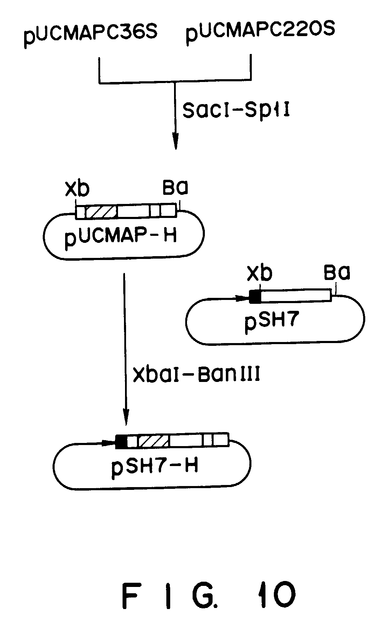

The present invention will be described in more detail by way of its examples. The present invention, however, is not limited to the following examples. In order to readily understand the present invention, steps A, B, C, and E are illustrated in Figs. 4, 5, 6, and 7, respectively, steps F and G are illustrated in Fig. 8, steps H and I are illustrated in Fig. 9, and steps J and K are illustrated in Fig. 10.

### A) A Step of Inserting A Synthetic DNA Fragment into Plasmid pKK223-3

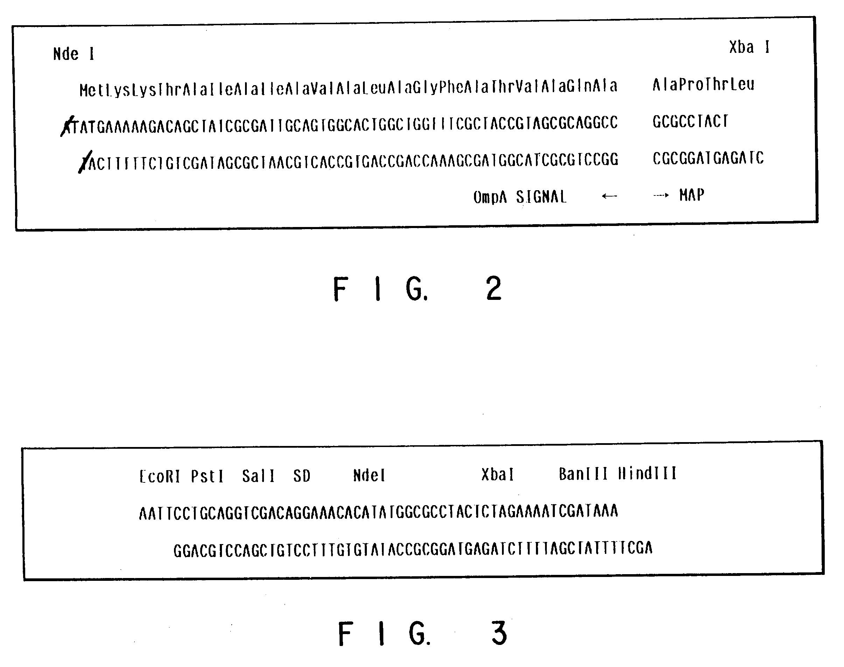

A DNA linker (SEQ ID No:7) coding restriction enzyme sites, SD sequence, methionine codon, and N-terminal amino acid sequence of MAP shown in Fig. 3 was inserted into plasmid pKK223-3 extracted from E.coli (strain HB101) transformed by a known method.

One micro gram of pKK223-3 was incubated in High Buffer (a mixture of 50mM Tris.HCℓ [pH7.5]-100mM of Nacℓ-1mM of MgCℓ) containing 10 units of each restriction enzyme of EcoRI and HindIII (manufactured by Nippon Gene Co, Ltd.) at 37°C for one hour for digestion. The obtained solution was subjected to phenol-chloroform treatment and ethanol precipitation to collect DNA. The phenol-chloroform treatment is described in detail was follows. Firstly, phenol was saturated with a mixture (hereinafter abbreviated as TE) of 10mM of Tris HCℓ (pH8.0) and 1mM of ethylenediamine tetraacetic acid (EDTA). The equivalent volume of the resultant phenol solution was added to the obtained DNA solution for mixing, and the resultant mixture was centrifuged to collect a aqueous phase containing DNA. Next, an equivalent volume of chloroform was added to this aqueous phase for further mixing, and the resultant mixture was centrifuged to collect an aqueous phase containing DNA. Ethanol precipitation is described in detail as follows. Firstly, to the obtained solution containing DNA, 5M of sodium chloride of 1/20-fold volume and ethanol of 2-fold volume were added, and the resultant mixture was cooled at -70°C for thirty minutes. Next, this solution was centrifuged at high speed to separate the obtained precipitant.

Two kind of single-stranded synthetic DNA linkers having base sequences shown in Fig. 3 and of complementary to each other were prepared by utilizing the DNA synthesizer (manufactured by Applied Biosystems Japan Company, 381A-type) according to the phosphoramidide method. One microgram of each obtained synthetic linker was incubated in 100 µℓ of a kinase solution (a mixture of 50mM of Tris HCℓ [pH7.6], 10mM of MgCℓ₂, 5mM of dithiothreitol, 0.1mM of spermidine, 0.1mM of EDTA, and 1mM of ATP) containing 10 unites of T4 kinase (manufactured by Toyobo Co., Ltd.) at 37°C for one hour to add phosphoric acid to 5'-terminal of the linkers. After that, the obtained single-stranded DNA was converted into double-stranded DNA by annealing. This annealing was performed by mixing the obtained reacted solutions, heating the resultant mixture at 60°C for twenty minutes, and allowing it to stand at room temperature for twenty minutes. Next, the resultant solution was subjected to ethanol precipitation, and then the precipitate was dissolved in 10 µℓ of TE.

To 5 µℓ of the thus obtained solution containing double-stranded synthetic DNA, 2.5 µℓ of (ca. 0.5 µg) of pKK223-3 cleavage product was added and the mixture was ligated at 10°C for two hours utilizing the ligation kit (manufactured by Takara Shuzo Co., Ltd). After that, the resultant DNA was utilized to transform E.coil (strain HB101). From the obtained transformants, plasmid pKS2 was prepared. This plasmid pKS2 is the plasmid formed by inserting the synthetic DNA linker into the plasmid pKK223-3.

### B) A Step of Converting Replication Origin of pKS2 to that of plasmid pUC19 Type

Two microgram of plasmid pKS2 and restriction enzyme BamHl were incubated at 37°C for one hour in 50 µℓ of High Buffer for digestion. Next, the obtained solution was subjected to phenol-chloroform treatment and ethanol precipitation to collect cleaved DNA. The obtained precipitate was added to 25 µℓ of Klenow solution (which is obtained by adding 0.1mM of each cofactor dATP, dGTP, dCTP and TTP to a mixture of 50mM of Tris.HCℓ [pH7.2], 10mM of MgS04, 0.1mM of dithiothreitol, and 50 µg/mℓ of bovine serum albumin) containing 2 units of Klenow fragment (manufactured by Toyobo Co., Ltd.) and the resultant solution was incubated at 22°C for 30 minutes to convert a cohesive end of the DNA to a flush end. After reaction, the solution was heated at 70°C for five minutes, followed by phenol-chloroform treatment and ethanol precipitation to collect DNA. The collected DNA was further cleaved by dissolving it in 50 µℓ of High Buffer containing 10 units of restriction enzyme PvuI (manufactured by Toyobo Co., Ltd.) and reacting at 37°C for one hour. The cleaved DNA was collected by phenol-chloroform treatment followed by ethanol precipitation.

Separately, 1 µg of plasmid pUC 19 (manufactured by Takara Syuzo Co., Ltd.) was incubated in 50 µℓ of High Buffer containing 10 units of each restriction enzyme PvuI (manufactured by Toyobo Co., Ltd.) and PvuII (manufactured by Nippon Gene Ltd.) at 37°C for two hours to cleave pUC19. The cleaved DNA fragments were collected by phenol-chloroform treatment, followed by ethanol precipitation.

The DNA fragment (larger fragment) derived from pKS2 and DNA fragment derived from pUC19 both of which were obtained as mentioned were dissolved in 10 µℓ of TE, respectively. After that, each 3.5 µℓ of the TE solutions were mixed and the fragments was ligated at 10°C for one hour utilizing the ligation kit (manufactured by Takara Shuzo Co., Ltd.). After that, the resultant DNA was utilized to transform E.coil (strain HB101). From the obtained transformants, plasmid pKS3 was prepared. This pKS3 comprises the replication origin of pUC19 and the large fragment of pKS2 which are combined therein.

### C) A Step of Inserting PL Promotor into pKS3

Two micrograms of pKS3 was incubated in 50 µℓ of High Buffer containing 10 units of a restriction enzyme PstI (manufactured by Nippon Gene Co., Ltd.) at 37°C for one hour for digestion. The cleaved DNA fragment was collected by phenol-chloroform treatment followed by ethanol precipitation. The collected DNA fragment was incubated in 20 µℓ of polymerase solution (which was obtained by adding 0.1mM of each co-factor of dATP, dGTP, dCTP and TTP to a mixture of 33mM of Tris·HCℓ [pH7.9], 66mM of potassium phosphate, 10mM of magnesium acetate, 0.5mM of dithiothreitol, and O.lmg/mℓ of bovine serum albumin) containing 2.5 units of T4 DNA polymerase (manufactured by Toyobo Co., Ltd.) at 37°C for five minutes to convert a cohesive end of the DNA fragment to a flush end. Next, 1 µℓ of 0.5M EDTA was added to the resultant solution, and the obtained mixture was subjected to phenol-chloroform treatment and further ethanol precipitation to collect DNA. The collected DNA was incubated in 50 µℓ of High Buffer containing 10 units of restriction enzyme BamHl (manufactured by Nippon Gene Co., Ltd.) at 37°C for one hour for additional digestion.

Separately, 1 µg of pPL-lambda (manufactured by Pharmacia Co., Ltd.) was incubated in 50 µℓ of High Buffer containing 10 units of each restriction enzyme BamHI and HpaI (both manufactured by Nippon Gene Co., Ltd.) at 37°C for one hour for digestion. Next, the mixture was subjected to phenol-chloroform treatment and the following ethanol precipitation to collect DNA fragment containing the P_{L} promotor.

The DNA fragment derived from pKS3 and the DNA fragment containing the P_{L} promoter were dissolved in 10 µℓ of TE, respectively. Next, 3.5 µℓ of each solution obtained as said were mixed, and the DNA fragments therein were ligated utilizing the ligation kit (manufactured by Takara Syuzo Co., Ltd.). After that, the resultant DNA was utilized to transform E.coli (strain HB101). From the obtained transformants, plasmid pSH4 was prepared. This plasmid pSH4 is the plasmid which was formed by inserting the P_{L} promotor into pKS3.

### D) A Step of Cleaving out cI857 Gene form Lambda-phage DNA

Two microgram of lambda-phage (lambda, cI₈₅₇, Sam7) DNA (manufactured by Takara Syuzo Co., Ltd.) was incubated in 50 µℓ of High Buffer containing 10 units of each restriction enzyme BglII (manufactured by Nippon Gene Co., Ltd.) and BanIII (manufactured by Toyobo Co., Ltd.) at 37°C for two hours for digestion. The obtained DNA fragments were collected by phenol-chloroform treatment followed by ethanol precipitation.

Meanwhile, 1 µg of plasmid pHSG397 (manufactured by Takara Syuzo Co., Ltd.) was incubated in 50 µℓ of High Buffer containing 10 units of each restriction enzyme BamHI (Nippon Gene Co., Ltd.) and BanIII (manufactured by Toyobo Co., Ltd.) at 37°C for one hour for digestion. Next, to the mixture, 2 µℓ (1 unit) of alkaline phosphatase (manufactured by Toyobo Co., Ltd.) was added, and the resultant mixture was allowed to heat at 60°C for 30 minutes for dephosphorylation at 5'-terminal of the DNA. After that, the DNA was collected by phenol-chloroform treatment and the following ethanol precipitation.

The lambda-phage DNA cleavage product and the pHSG397 cleavage product both of which were thus obtained were dissolved in each 10 µℓ of TE. Next, 3.5 µℓ of each solution were mixed and the DNA cleavage products therein were ligated by reacting the mixture at 10°C for two hours utilizing the ligation kit (manufactured by Takara Syuzo Co., Ltd.). The obtained DNA was utilized to transform E.coli (strain HB101), and plasmid DNA was purified from the obtained transformants. This plasmid DNA is pHSGcI₈₅₇ formed by inserting BglII-BanIII fragment of ca.1100 base pairs including cI₈₅₇ into pHSG397.

### E) A Step of Inserting cI₈₅₇ into pSH4

Two micrograms of pHSGcI₈₅₇ was incubated in 50 µℓ of High Buffer containing 10 units of each restriction enzyme XhoI (manufactured by Nippon Gene Co., Ltd.) and BanIII (manufactured by Toyobo Co., Ltd.) at 37°C for one hour for digestion. The cleaved DNA fragments were collected by phenol-chloroform treatment and the following ethanol precipitation. The collected DNA was incubated in 25 µℓ of Klenow solution containing two units of Klenow fragment at 22°C for 30 minutes to convert a cohesive end of the DNA to a flush end. Next, the resultant solution was heated at 70°C for five minutes and subjected to phenol-chloroform treatment and the following ethanol precipitation to collect the DNA.

On the other hand, 1 µg of pSH4 was incubated in 50 µℓ of High Buffer containing 10 units of restriction enzyme BamHl (manufactured by Nippon Gene Co., Ltd.) at 37°C for one hour for digestion. The cleaved DNA was collected by phenol-chloroform treatment and the following ethanol precipitation. The collected DNA was incubated in 25 µℓ of Klenow solution containing 2 units of Klenow fragment at 22°C for 30 minutes to convert a cohesive end of the DNA to a flush end. Next, the resultant solution was heated at 70°C for five minutes, and subjected to phenol-chloroform treatment and the following ethanol precipitation to collect the resultant DNA.

The DNA fragment including cI₈₅₇ and the cleaved pSH4 fragments both of which were thus obtained were dissolved in 10 µℓ of TE, respectively. Next, 3.5 µℓ of each solution was mixed and the DNA fragments were ligated by reacting the solution at 10°C for two hours utilizing the ligation kit (manufactured by Takara Syuzo Co., Ltd.). The obtained DNA was utilized to transform E.coli (strain HB101), and plasmid DNA was purified from the obtained transformants. The obtained plasmid is pSH5 formed by inserting cI₈₅₇ into plasmid pSH4.

### F) A Step of Inserting a Total Synthetic MAP Gene into pSH5

Two micrograms of pSH5 was incubated in 50 µℓ of High Buffer containing 10 units of each restriction enzyme XbaI (manufactured by Nippon Gene Co., Ltd.) and BanIII (manufactured by Toyobo Co., Ltd.) at 37°C for one hour for digestion. The resultant solution was subjected to phenol-chloroform treatment and the following ethanol precipitation to collect the cleaved DNA.

Meanwhile, 2 µg of pMHI was digested and the cleaved DNA was collected by the same manner as said for pSH5. The pMHI here is a synthetic plasmid formed by inserting a total synthetic MAP gene into the plasmid pUC19 and extracted from above mentioned E.coli transformant (deposit No. 9913) deposited in FIR.

The DNA fragment derived from pSH5 and the fragment from pMHI thus obtained were dissolved into 10 µℓ of each TE, respectively. Next, 3.5 µℓ of each solution was mixed and the DNA fragments therein were ligated by reacting the resultant mixture at 10°C for one hour utilizing the ligation kit (manufactured by Takara Syuzo Co., Ltd.). The combined DNA was used to transform E.coli (strain N99cI+), and plasmid DNA was purified from the obtained transformants. This plasmid is pSH6 formed by inserting a fragment of the total synthetic MAP gene, which obtained by cleaving with XbaI and BanIII, into the plasmid pHS5.

### G) A Step of Inserting Signal Sequence Gene of OmpA into pSH6

Each single-stranded DNA of complementary DNA fragments having a base sequences shown in Fig. 2 was synthesized according to phosphoramidide method utilizing a DNA synthesizer (manufactured by Applied Biosystems Japan, 381A type). One microgram of each synthesized single-stranded DNA was incubated in 50 µℓ of said kinase solution containing 10 units of T4 kinase (manufactured by Toyobo Co., Ltd.) at 37°C for one hour to phosphorylate 5'-terminal of the DNA. Each solution containing the phosphorylated single-stranded DNA were mixed and the resultant solution was heated at 60°C for 20 minutes and allowed to stand at room temperature for 20 minutes for annealing to obtain double-stranded DNA. The obtained double-stranded DNA was collected by ethanol precipitation and dissolved in 10 µℓ of TE.

On the other hand, 1 µg of the plasmid pSH6 was incubated in 50 µℓ of High Buffer containing 10 units of each restriction enzyme NdeI and XbaI (both manufactured by Nippon Gene Co., Ltd.) at 37°C for one hour for digestion. The cleaved DNA was collected by subjecting the reacted solution to phenol-chloroform treatment and the following ethanol precipitation. The collected DNA was further dissolved in 10 µℓ of TE.

Three point five micro liter of the TE solution containing the annealed synthetic DNA and also 3.5 µℓ of the TE solution containing the cleaved pSH6 was mixed. The mixture was reacted at 10°C for two hours by utilizing the ligation kit (manufactured by Takara Syuzo Co., Ltd.) to combine the synthetic DNA and the cleaved DNA. The combined DNA was used to transform E.coli (strain N99cI+), and plasmid DNA was purified from the obtained transformants. This plasmid is plasmid pSH7 formed by inserting the gene of OmpA signal sequence into pSH6.

### H) A step of Cloning of Blocks II and VII of MAP Variant

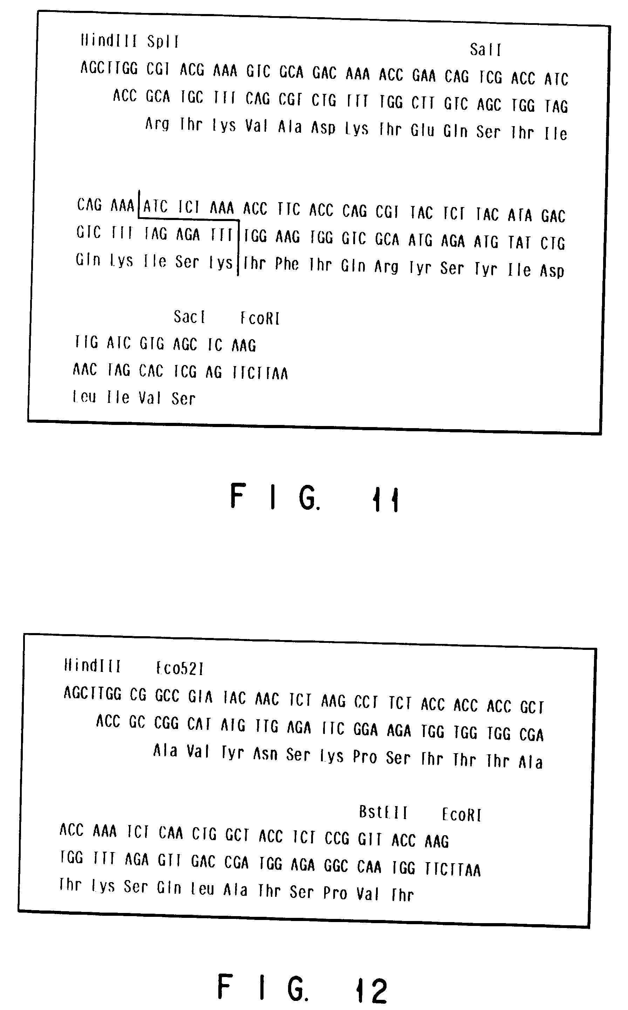

DNA fragments having a base sequence (SEQ ID No:3) shown in Fig. 11 as four fragments and DNA fragments having a base sequence (SEQ ID NO:4) shown in Fig. 12 as two fragments were synthesized by DNA synthesizer (manufactured by Applied Biosystems Japan Company, 381A-type), respectively. The synthesized products are DNA fragments of blocks II and VII of MAP-H genes. In this case, codons for coding serines in place of those for coding cysteines were used as "TCG" and "TCT", respectively. However, the codons are not limited to the above. Any codon may be used if it codes serine.

One microgram of each fragment was incubated in 50 µℓ of a kinase solution containing 10 units of T4 kinase (manufactured by Toyobo Co., Ltd.) at 37°C for one hour to phosphorylate its 5'-terminal. The four kinds of incubated solutions each containing different fragments were mixed for block II, and the two kinds of incubated solutions each containing different fragments were mixed for block VII at 60°C for 20 minutes, and the resultant solutions were left to stand for an hour at room temperature, thereby annealing complementary chains. Five µℓ of each resultant solution were added to 2.5 µℓ of a solution containing 0.1 µg of DNA obtained by cleaving plasmid pUC 19 with restriction enzymes EcoRI and HindIII in advance, treating with phenol and precipitating with ethanol, and were linked using the commercially available ligation kit (manufactured by Takara Shuzo Co., Ltd.). E.coli of strain HB101 was transformed by the calcium method using the resultant plasmid, and the plasmid was purified from the resultant transformant by the alkaline SDS method, thereby confirming that the synthesized DNA fragments were inserted. This confirmation was performed using a sequence determination for DNA according to the dideoxy method. Plasmid pUCMBC36S was obtained by insertion of fragments of block II, and plasmid pUCMBC220S was obtained by insertion of fragments of block VII.

### I) A step of Inserting Blocks II and VII into A Complete Synthetic MAP Gene

Block II was cleaved out from the resultant plasmid pUCMBC36S by restriction enzymes SacI and SplI, and block VII was cleaved out from the resultant plasmid pUCMBC220S by restriction enzymes Eco521 and BstEII. The cleaved blocks are inserted into the corresponding blocks of the complete synthetic MAP gene and thus substitute tam as follows. About 1 µg of each DNA cleaved by the corresponding restriction enzymes was subjected to phenol treatment and ethanol precipitation and was dissolved in 10 µℓ of a solution containing 10mM Tris·HCℓ [pH8.0] and 10mM EDTA, respectively, 3.5 µℓ of each resultant solution were mixed, and the DNA contained in each solution was linked to each other by the ligation kit (manufactured Takara Shuzo Co., Ltd.). The complete MAP gene was extracted from the E.coli transformant deposited in FRI as deposit No. 9913. Using the resultant plasmid, i.e., a plasmid obtained by inserting a small fragment (i.e., block II or VII) cleaved out from the plasmid pUCMBC36S or pUCMBC220S into the complete synthetic MAP gene (pMH1), E.coli (strain HB101) was transformed by the calcium method. The plasmid was purified by the alkaline-SDS method from the resultant transformant. Insertion of each block was confirmed by the presence of a newly introduced SalI site for block II and by the absence of a deleted PvuI site for block VII. Plasmid pUCMAPC36S was obtained upon insertion of block II, and plasmid pUCMAPC220S was obtained upon insertion of block VII.

### J) A step of producing MAP Gene in which Two Codons for coding Cystines are Substituted with Codons for coding Serines

Two plasmids pUCMAPC36S and pUCMAPC220S were cleaved by restriction enzymes SacI and SpII. The block II obtained by cleaving plasmid pUCMAPC36S was further cleaved by restriction enzyme ScaI so as not to convert it into the original plasmid. In a sample containing block VII obtained by cleaving plasmid pUCMAPC220S, alkaline phosphates (manufactured by Toyobo Co., Ltd.) was added to the reacted solution to eliminate phosphoric acid at its 5'-terminal, so that the resultant DNA was not converted into the original plasmid unless other DNAs were inserted at positions cleaved by the restriction enzymes.

The respective plasmid decomposed products were subjected to phenol treatment and ethanol precipitation. Each sample was then dissolved in 10 µℓ of a solution containing 10 mM Tris·HCℓ [pH8.0] and 10 mM EDTA, and each 3.5 µℓ of the resultant solutions were mixed to link them using the ligation kit (manufactured by Takara Shuzo Co., Ltd.) E.coli (strain MV1184) was transformed using the resultant plasmid in which both blocks II and VII were inserted. The plasmid was purified by the alkaline SDS method from the resultant transformant. Insertion was confirmed by the presence/absence of the restriction enzyme site as in the previous step, thereby obtaining plasmid pUCMAP-H. This plasmid contains the MAP-H gene having a base sequence represented as SEQ ID No:2 in the Sequence Listing.

### K) A step of Inserting MAP-H Gene Having A Base Sequence into MAP Secretion Expression Vector pSH7

The plasmid pUCMAP-H obtained in step J and the MAP expression secretion vector pSH7 obtained in step G were cleaved by restriction enzymes XbaI and BanIII, respectively, and were subjected to phenol treatment and ethanol precipitation. Each sample was dissolved in 10 µℓ of a solution containing 10 mM Tris·HCℓ [pH 8.0] and 10 mM EDTA, and each 3.5 µℓ of the resultant solutions was mixed and linked using the ligation kit (manufactured by Takara Shuzo Co., Ltd.) E.coli (strain 99cI+) was transformed by the calcium method using the resultant plasmid. The plasmid was then purified from the resultant transformant by the alkaline-SDS method, thereby obtaining plasmid pSH7H in which the MAP-H gene was inserted in pSH7. This E.coli transformant having plasmid pSH7H has already been deposited in FRI (deposit No. :FRI No. 12093).

### L) A Step of Producing and Purificating A MAP Variant Having No Cysteine Bond

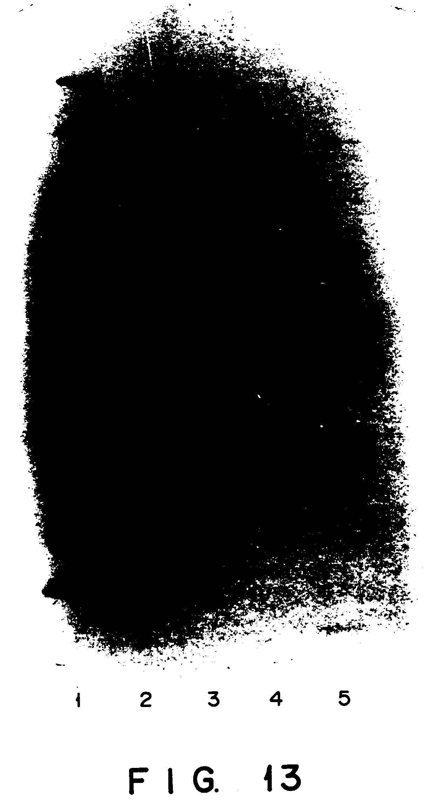

E.coli (strain MM294) was transformed by the calcium method using secretion vector pSH7H in which the MAP-H gene was inserted. The resultant transformant was subjected to a shaking cultuer in 2ℓ of an L medium (a mixture of 1% bact.trypton, 0.5% bact.yeast extract, 0.5% sodium chloride, and 0.1% glucose) at 30°C. When absorption of the medium solution at 550 nm was set to be 0.8, a medium preheated at 55°C in an equivalent amount was added to the above medium, and the temperature of the total medium was set at 42°C. A shaking culture was continued at this temperature for 3 hours. Thereafter, ammonium sulfate was added to the medium from which bacterial body had been eliminated by centrifugal separation, so that a concentration of ammonium sulfate was set in a 90% to saturation, and the protein was salted out. This protein was precipitated by centrifugal separation and was collected. The collected protein was dissolved in 40 mℓ of A buffer solution (10mM sodium phosphate buffer solution [pH6.0]), and the A buffer solution was dialyzed. The resultant crude extract was subjected on a Carboxymethyl Sepharose column (26 mm in diameter and 40 mm in length) pretreated with the A buffer solution. After the column was sufficiently washed with the A buffer solution, the adsorbed protein was eluted with a sodium chloride solution having linear gradient of 0M to 0.5M. The MAP-H fractions of the eluted protein were collected by the ELISA using an anti-MAP antiserum. The collected proteins were dialyzed using a B buffer solution (10mM Tris/hydrochloricde buffer solution [pH8.0]) and were subjected on a Blue Sepharose column (5 mm in diameter and 50 mm in length) pretreated with the B buffer solution. After the column was sufficiently washed with the B buffer solution, and the protein was eluted with a sodium chloride solution containing linear gradient of 0M to 0.2M. MAP-H fractions of the eluted protein were collected by the ELISA using an anti-MAP antiserum and were dialyzed using distilled water, thereby purifying MAP-H. This MAP-H was analyzed by SDS-polyacrylamide gel electrophoresis, and the result is shown in Fig. 13. Referring to Fig. 13, lane 1 represents molecular weight markers, i.e., each band represents 97, 66, 42, 30, and 20 kilodaltons from the above, respectively. Lanes 2 to 4 represent natural MAPs, and lane 5 represents MAP-H of the present invention. The MAP in lane 2 is reduced with 2-mercaptoethanol immediately before electrophoresis to cleave an S-S bond. As compared with the MAP in lane 4, which not subjected to reduction, a pattern is found to be changed (a and b). The MAP-H in lane 5 shows the same pattern as the MAP in which the S-S bond is cleaved. Almost no bands representing other impurities are found in the MAP-H migration patterns shown in Fig. 13, and the protein is found to be almost uniform.

### M) Inhibition Effect of MAP-H to in Vitro Protein Synthesis

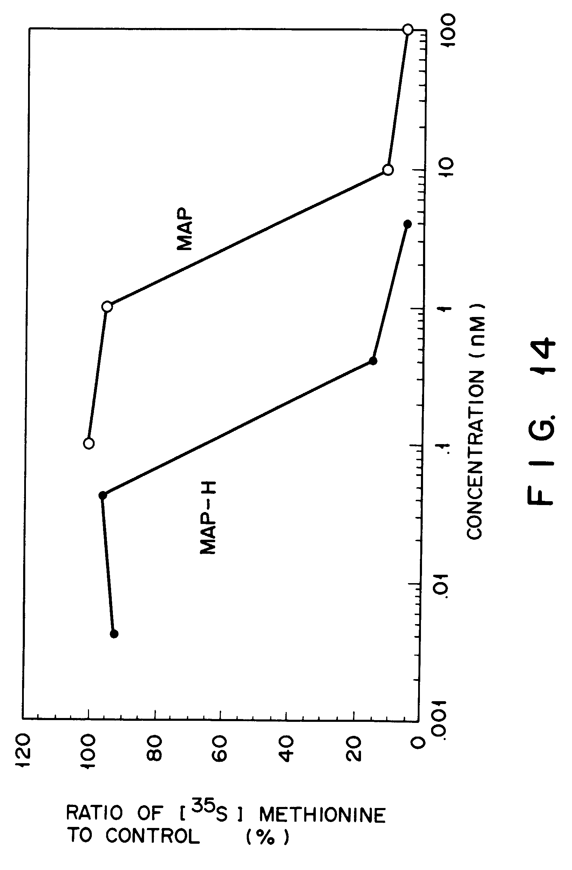

Tobacco mosaic virus RNA as mRNA was added to 10 µℓ of a commercially available rabbit reticulocyte crude extract containing ³⁵S methionine and an appropriate amount of MAP or MAP-H and the resultant mixture was incubated for translation at 30°C for 30 minutes. Two µℓ of this reacted solution were taken on filter paper and dried, and this paper was boiled in a 10% trichloroacetic acid solution for 10 minutes. The radioactivity of a radioactive substance (polypeptide containing ³⁵S incorporated) left on the filter paper was measured using a toluene-based scintillator. The radioactivity of a sample not containing the mRNA was defined as 0%, and the radioactivity of a sample not containing the MAP or MAP-H was defined as 100% as a control, and influences of the MAP and MAP-H were examined. Results are shown in Fig. 14. Referring to Fig. 14, a ratio of an amount of incorporated ³⁵S to the control is plotted along the ordinate, and the MAP or MAP-H concentration is plotted along the abscissa.

As is apparent from Fig. 14, the MAP in about 3.5 mM exhibited a 50% inhibition effect in this system, and the MAP-H in about 0.16 nM exhibited a 50% inhibition effect. That is, the activity of protein synthesis inhibition of MAP-H was improved about 22 times that of the natural MAP.

As has been described above in detail, the MAP variant (MAP-H) having no S-S bond and serving as an antiviral protein according to the present invention has a higher activity of a protein synthesis inhibition than that of the natural protein while preserving the advantages of the natural protein. Therefore, the antiviral protein according to the present invention is most promising as a toxic protein used as, e.g., an immunotoxin.

SEQ ID NO:1

SEQUENCE LENGTH:250

SEQUENCE TYPE:amino acid

MOLECULE TYPE:protein

HYPOTHETICAL:Yes

ANTI-SENSE:No

FRAGMENT TYPE:internal fragment

Ala Pro Thr Leu Glu Thr Ile Ala Ser Leu Asp Leu Asn Asn Pro

1               5                   10                  15

Thr Thr Tyr Leu Ser Phe Ile Thr Asn Ile Arg Thr Lys Val Ala

                20                  25                  30

Asp Lys Thr Glu Gln Ser Thr Ile Gln Lys Ile Ser Lys Thr Phe

                35                  40                  45

Thr Gln Arg Tyr Ser Tyr Ile Asp Leu Ile Val Ser Ser Thr Gln

                50                  55                  60

Lys Ile Thr Leu Ala Ile Asp Met Ala Asp Leu Tyr Val Leu Gly

                65                  70                  75

Tyr Ser Asp Ile Ala Asn Asn Lys Gly Arg Ala Phe Phe Phe Lys

                80                  85                  90

Asp Val Thr Glu Ala Val Ala Asn Asn Phe Phe Pro Gly Ala Thr
                95                  100                 105
Gly Thr Asn Arg Ile Lys Leu Thr Phe Thr Gly Ser Tyr Gly Asp
            110                 115                 120
Leu Glu Lys Asn Gly Gly Leu Arg Lys Asp Asn Pro Leu Gly Ile
            125                 130                 135
Phe Arg Leu Glu Asn Ser Ile Val Asn Ile Tyr Gly Lys Ala Gly
            140                 145                 150
Asp Val Lys Lys Gln Ala Lys Phe Phe Leu Leu Ala Ile Gln Met
            155                 160                 165
Val Ser Glu Ala Ala Arg Phe Lys Tyr Ile Ser Asp Lys Ile Pro
            170                 175                 180
Ser Glu Lys Tyr Glu Glu Val Thr Val Asp Glu Tyr Met Thr Ala
            185                 190                 195
Leu Glu Asn Asn Trp Ala Lys Leu Ser Thr Ala Val Tyr Asn Ser
            200                 205                 210
Lys Pro Ser Thr Thr Thr Ala Thr Lys Ser Gln Leu Ala Thr Ser
            215                 220                 225

Pro Val Thr Ile Ser Pro Trp Ile Phe Lys Thr Val Glu Glu Ile
                230                 235                 240
Lys Leu Val Met Gly Leu Leu Lys Ser Ser
                245                 250

SEQ ID NO:2
SEQUENCE LENGTH:762
SEQUENCE TYPE:nucleic acid
STRANDEDNESS:double
TOPOLOGY:linear
MOLECULE TYPE:Other nucleic acid, synthetic DNA
HYPOTHETICAL:Yes
ANTI-SENSE:No

GCG CCT ACT CTA GAA ACC ATC GCT TCT CTG GAC CTG AAC AAC CCG 45
Ala Pro Thr Leu Glu Thr Ile Ala Ser Leu Asp Leu Asn Asn Pro
1               5                   10                  15
ACC ACC TAC CTG TCT TTC ATA ACG AAT ATC CGT ACG AAA GTC GCA 90
Thr Thr Tyr Leu Ser Phe Ile Thr Asn Ile Arg Thr Lys Val Ala
                20                  25                  30

GAC AAA ACC GAA CAG TCG ACC ATC CAG AAA ATC TCT AAA ACC TTC 135
Asp Lys Thr Glu Gln Ser Thr Ile Gln Lys Ile Ser Lys Thr Phe
                35                  40                  45
ACC CAG CGT TAC TCT TAC ATA GAC TTG ATC GTG AGC TCG ACG CAG 180
Thr Gln Arg Tyr Ser Tyr Ile Asp Leu Ile Val Ser Ser Thr Gln
                50                  55                  60
AAA ATC ACC CTA GCT ATC GAC ATG GCT GAC CTG TAC GTT CTG GGT 225
Lys Ile Thr Leu Ala Ile Asp Met Ala Asp Leu Tyr Val Leu Gly
                65                  70                  75
TAC TCT GAC ATC GCT AAT AAC AAG GGT CGT GCT TTC TTC TTC AAA 270
Tyr Ser Asp Ile Ala Asn Asn Lys Gly Arg Ala Phe Phe Phe Lys
                80                  85                  90
GAC GTG ACT GAG GCT GTT GCG AAC AAT TTC TTC CCG GGA GCT ACA 315
Asp Val Thr Glu Ala Val Ala Asn Asn Phe Phe Pro Gly Ala Thr
                95                  100                 105
GGT ACT AAT CGT ATC AAA TTA ACC TTT ACA GGT TCT TAT GGC GAT 360
Gly Thr Asn Arg Ile Lys Leu Thr Phe Thr Gly Ser Tyr Gly Asp
                110                 115                 120
CTC GAG AAA AAC GGC GGA CTA CGT AAG GAC AAT CCC CTA GGT ATC 405

Leu Glu Lys Asn Gly Gly Leu Arg Lys Asp Asn Pro Leu Gly Ile
                125                 130                 135

TTC CGT CTG GAA AAC TCG ATA GTT AAC ATT TAT GGC AAA GCT GGT 450
Phe Arg Leu Glu Asn Ser Ile Val Asn Ile Tyr Gly Lys Ala Gly
                140                 145                 150

GAC GTT AAA AAA CAG GCT AAA TTC TTC TTA CTG GCT ATC CAG ATG 495
Asp Val Lys Lys Gln Ala Lys Phe Phe Leu Leu Ala Ile Gln Met
                155                 160                 165

GTT TCG GAG GCT GCG CGC TTT AAG TAT ATC AGT GAC AAA ATC CCG 540
Val Ser Glu Ala Ala Arg Phe Lys Tyr Ile Ser Asp Lys ILe Pro
                170                 175                 180

TCT GAA AAA TAC GAA GAA GTT ACC GTT GAC GAA TAC ATG ACC GCT 585
Ser Glu Lys Tyr Glu Glu Val Thr Val Asp Glu Tyr Met Thr Ala
                185                 190                 195

CTG GAA AAC AAC TGG GCT AAA CTG TCT ACG GCC GTA TAC AAC TCT 630
Leu Glu Asn Asn Trp Ala Lys Leu Ser Thr Ala Val Tyr Asn Ser
                200                 205                 210

AAG CCT TCT ACC ACC ACC GCT ACC AAA TCT CAA CTG GCT ACC TCT 675

Lys Pro Ser Thr Thr Thr Ala Thr Lys Ser Gln Leu Ala Thr Ser
            215                 220                 225
CCG GTT ACC ATC TCT CCG TGG ATA TTC AAA ACC GTC GAG GAA ATC     720
Pro Val Thr Ile Ser Pro Trp Ile Phe Lys Thr Val Glu Glu Ile
            230                 235                 240
AAA CTG GTT ATG GGT CTG CTT AAG TCT TCT TAATAAATCG AT          762
Lys Leu Val Met Gly Leu Leu Lys Ser Ser
            245                 250

SEQ ID NO:3
SEQUENCE LENGTH:108
SEQUENCE TYPE:nucleic acid
STRANDEDNESS:both
TOPOLOGY:linear
MOLECULE TYPE:Other nucleic acid, synthetic DNA
HYPOTHETICAL:Yes
ANTI-SENSE:No
FEATURE:
OTHER INFORMATION:Base sequence corresponding to 76-173 base pairs of a sequence as
shown in SEQ ID NO:2

AGCTTGG CGT ACG AAA GTC GCA GAC AAA ACC GAA CAG TCG ACC ATC 46
Arg Thr Lys Val Ala Asp Lys Thr Glu Gln Ser Thr Ile
1               5                   10
CAG AAA ATC TCT AAA ACC TTC ACC CAG CGT TAC TCT TAC ATA GAC 91
Gln Lys Ile Ser Lys Thr Phe Thr Gln Arg Tyr Ser Tyr Ile Asp
    15                  20                  25
TTG ATC GTG AGC TCAAG 108
Leu Ile Val Ser
    30
SEQ ID NO:4
SEQUENCE LENGTH:81
SEQUENCE TYPE:nucleic acid
STRANDEDNESS:both
TOPOLOGY:linear
MOLECULE TYPE:Other nucleic acid, synthetic DNA
HYPOTHETICAL:Yes
ANTI-SENSE:No
FEATURE:

OTHER INFORMATION:Base sequence corresponding to 614[?] 684 base pairs of a sequence
as shown in SEQ ID NO:2
AGCTTGGCG GCC GTA TAC AAC TCT AAG CCT TCT ACC ACC GCT 45
Ala Val Tyr Asn Ser Lys Pro Ser Thr Thr Ala
1               5                   10
ACC AAA TCT CAA CTG GCT ACC TCT CCG GTT ACC AAG 81
Thr Lys Ser Gln Leu Ala Thr Ser Pro Val Thr
            15                  20
SEQ ID NO:5
SEQUENCE LENGTH:756
SEQUENCE TYPE:nucleic acid
STRANDEDNESS:double
TOPOLOGY:linear
MOLECULE TYPE:Other nucleic acid, synthetic DNA
GCG CCT ACT CTA GAA ACC ATC GCT TCT CTG GAC CTG AAC AAC CCG 45
ACC ACC TAC CTG TCT TTC ATA ACG AAT ATC CGT ACG AAA GTC GCA 90
GAC AAA ACC GAA CAG TGT ACC ATC CAG AAA ATC TCT AAA ACC TTC 135
ACC CAG CGT TAC TCT TAC ATA GAC TTG ATC GTG AGC TCG ACG CAG 180

AAA ATC ACC CTA GCT ATC GAC ATG GCT GAC CTG TAC GTT CTG GGT 225

TAC TCT GAC ATC GCT AAT AAC AAG GGT CGT GCT TTC TTC TTC AAA 270

GAC GTG ACT GAG GCT GTT GCG AAC AAT TTC TTC CCG GGA GCT ACA 315

GGT ACT AAT CGT ATC AAA TTA ACC TTT ACA GGT TCT TAT GGC GAT 360

CTC GAG AAA AAC GGC GGA CTA CGT AAG GAC AAT CCC CTA GGT ATC 405

TTC CGT CTG GAA AAC TCG ATA GTT AAC ATT TAT GGC AAA GCT GGT 450

GAC GTT AAA AAA CAG GCT AAA TTC TTC TTA CTG GCT ATC CAG ATG 495

GTT TCG GAG GCT GCG CGC TTT AAG TAT ATC AGT GAC AAA ATC CCG 540

TCT GAA AAA TAC GAA GAA GTT ACC GTT GAC GAA TAC ATG ACC GCT 585

CTG GAA AAC AAC TGG GCT AAA CTG TCT ACG GCC GTA TAC AAC TCT 630

AAG CCT TCT ACC ACC ACC GCT ACC AAA TGT CAG CTG GCT ACC TCT 675

CCG GTT ACC ATC TCT CCG TGG ATA TTC AAA ACC GTC GAG GAA ATC 720

AAA CTG GTT ATG GGT CTG CTT AAG TCT TCT TAATAA 756

SEQ ID NO:6

SEQUENCE LENGTH:74

SEQUENCE TYPE:nucleic acid

AT ATG AAA AAG ACA GCT ATC GCG ATT GCA GTG GCA CTG GCT GGT TTC 47
Met Lys Lys Thr Ala Ile Ala Ile Ala Val Ala Leu Ala Gly Phe
1               5                   10                  15
GCT ACC GTA GCG CAG GCC GCGCCTACT 74
Ala Thr Val Ala Gln Ala
                20

SEQ ID NO:7
SEQUENCE LENGTH:56
SEQUENCE TYPE:nucleic acid
AATTCCTGCAGGTCGACAGGAAACACATATGGCGCCTACTCTAGAAAATCGATAAA 56

## Claims

1. An antiviral protein having the following amino acid sequence:

Ala Pro Thr Leu Glu Thr Ile Ala Ser Leu Asp Leu Asn Asn Pro
1               5                   10                  15
Thr Thr Tyr Leu Ser Phe Ile Thr Asn Ile Arg Thr Lys Val Ala
                20                  25                  30
Asp Lys Thr Glu Gln Ser Thr Ile Gln Lys Ile Ser Lys Thr Phe
                35                  40                  45
Thr Gln Arg Tyr Ser Tyr Ile Asp Leu Ile Val Ser Ser Thr Gln
                50                  55                  60
Lys Ile Thr Leu Ala Ile Asp Met Ala Asp Leu Tyr Val Leu Gly
                65                  70                  75
Tyr Ser Asp Ile Ala Asn Asn Lys Gly Arg Ala Phe Phe Phe Lys
                80                  85                  90
Asp Val Thr Glu Ala Val Ala Asn Asn Phe Phe Pro Gly Ala Thr
                95                  100                 105
Gly Thr Asn Arg Ile Lys Leu Thr Phe Thr Gly Ser Tyr Gly Asp
                110                 115                 120
Leu Glu Lys Asn Gly Gly Leu Arg Lys Asp Asn Pro Leu Gly Ile
                125                 130                 135
Phe Arg Leu Glu Asn Ser Ile Val Asn Ile Tyr Gly Lys Ala Gly
                140                 145                 150
Asp Val Lys Lys Gln Ala Lys Phe Phe Leu Leu Ala Ile Gln Met
                155                 160                 165
Val Ser Glu Ala Ala Arg Phe Lys Tyr Ile Ser Asp Lys Ile Pro
                170                 175                 180
Ser Glu Lys Tyr Glu Glu Val Thr Val Asp Glu Tyr Met Thr Ala
                185                 190                 195
Leu Glu Asn Asn Trp Ala Lys Leu Ser Thr Ala Val Tyr Asn Ser
                200                 205                 210
Lys Pro Ser Thr Thr Thr Ala Thr Lys Ser Gln Leu Ala Thr Ser
                215                 220                 225
Pro Val Thr Ile Ser Pro Trp Ile Phe Lys Thr Val Glu Glu Ile
                230                 235                 240

Lys Leu Val Met Gly Leu Leu Lys Ser Ser
                245                 250

2. A gene encoding the protein as defined in claim 1.

3. A gene according to claim 2, which has the following base sequence:

GCG CCT ACT CTA GAA ACC ATC GCT TCT CTG GAC CTG AAC AAC CCG
ACC ACC TAC CTG TCT TTC ATA ACG AAT ATC CGT ACG AAA GTC GCA
GAC AAA ACC GAA CAG TCG ACC ATC CAG AAA ATC TCT AAA ACC TTC
ACC CAG CGT TAC TCT TAC ATA GAC TTG ATC GTG AGC TCG ACG CAG
AAA ATC ACC CTA GCT ATC GAC ATG GCT GAC CTG TAC GTT CTG GGT
TAC TCT GAC ATC GCT AAT AAC AAG GGT CGT GCT TTC TTC TTC AAA
GAC GTG ACT GAG GCT GTT GCG AAC AAT TTC TTC CCG GGA GCT ACA
GGT ACT AAT CGT ATC AAA TTA ACC TTT ACA GGT TCT TAT GGC GAT
CTC GAG AAA AAC GGC GGA CTA CGT AAG GAC AAT CCC CTA GGT ATC
TTC CGT CTG GAA AAC TCG ATA GTT AAC ATT TAT GGC AAA GCT GGT
GAC GTT AAA AAA CAG GCT AAA TTC TTC TTA CTG GCT ATC CAG ATG
GTT TCG GAG GCT GCG CGC TTT AAG TAT ATC AGT GAC AAA ATC CCG
TCT GAA AAA TAC GAA GAA GTT ACC GTT GAC GAA TAC ATG ACC GCT
CTG GAA AAC AAC TGG GCT AAA CTG TCT ACG GCC GTA TAC AAC TCT
AAG CCT TCT ACC ACC ACC GCT ACC AAA TCT CAA CTG GCT ACC TCT
CCG GTT ACC ATC TCT CCG TGG ATA TTC AAA ACC GTC GAG GAA ATC
AAA CTG GTT ATG GGT CTG CTT AAG TCT TCT TAA TAA ATC GAT

4. A recombinant plasmid comprising the gene as defined in claim 3, said gene being integrated in said plasmid.

5. A recombinant plasmid comprising the gene as defined in claim 3, said gene being inserted into a MAP gene region of plasmid pSH7, wherein said gene is integrated so that said gene is expressed in E.coli, and the protein produced is secreted.

6. E.coli transformed with the plasmid as defined in claim 5.

7. An antiviral protein obtainable by culturing the E.coli transformant as defined in claim 6, expressing the gene as defined in claim 3 to produce said protein, and secreting the said protein into a medium.

## Patentansprüche

1. Antivirales Protein mit der nachfolgenden Aminosäuresequenz:

Ala Pro Thr Leu Glu Thr Ile Ala Ser Leu Asp Leu Asn Asn Pro
1               5                   10                  15
Thr Thr Tyr Leu Ser Phe Ile Thr Asn Ile Arg Thr Lys Val Ala
                20                  25                  30
Asp Lys Thr Glu Gln Ser Thr Ile Gln Lys Ile Ser Lys Thr Phe
                35                  40                  45
Thr Gln Arg Tyr Ser Tyr Ile Asp Leu Ile Val Ser Ser Thr Gln
                50                  55                  60
Lys Ile Thr Leu Ala Ile Asp Met Ala Asp Leu Tyr Val Leu Gly
                65                  70                  75
Tyr Ser Asp Ile Ala Asn Asn Lys Gly Arg Ala Phe Phe Phe Lys
                80                  85                  90
Asp Val Thr Glu Ala Val Ala Asn Asn Phe Phe Pro Gly Ala Thr
                95                  100                 105
Gly Thr Asn Arg Ile Lys Leu Thr Phe Thr Gly Ser Tyr Gly Asp
                110                 115                 120
Leu Glu Lys Asn Gly Gly Leu Arg Lys Asp Asn Pro Leu Gly Ile
                125                 130                 135
Phe Arg Leu Glu Asn Ser Ile Val Asn Ile Tyr Gly Lys Ala Gly
                140                 145                 150
Asp Val Lys Lys Gln Ala Lys Phe Phe Leu Leu Ala Ile Gln Met
                155                 160                 165
Val Ser Glu Ala Ala Arg Phe Lys Tyr Ile Ser Asp Lys Ile Pro
                170                 175                 180
Ser Glu Lys Tyr Glu Glu Val Thr Val Asp Glu Tyr Met Thr Ala
                185                 190                 195
Leu Glu Asn Asn Trp Ala Lys Leu Ser Thr Ala Val Tyr Asn Ser
                200                 205                 210
Lys Pro Ser Thr Thr Thr Ala Thr Lys Ser Gln Leu Ala Thr Ser
                215                 220                 225
Pro Val Thr Ile Ser Pro Trp Ile Phe Lys Thr Val Glu Glu Ile
                230                 235                 240

Lys Leu Val Met Gly Leu Leu Lys Ser Ser
                245                 250

2. Gen, das für das Protein nach Anspruch 1 kodiert.

3. Gen nach Anspruch 2, mit der nachfolgenden Basensequenz:

GCG CCT ACT CTA GAA ACC ATC GCT TCT CTG GAC CTG AAC AAC CCG
ACC ACC TAC CTG TCT TTC ATA ACG AAT ATC CGT ACG AAA GTC GCA
GAC AAA ACC GAA CAG TCG ACC ATC CAG AAA ATC TCT AAA ACC TTC
ACC CAG CGT TAC TCT TAC ATA GAC TTG ATC GTG AGC TCG ACG CAG
AAA ATC ACC CTA GCT ATC GAC ATG GCT GAC CTG TAC GTT CTG GGT
TAC TCT GAC ATC GCT AAT AAC AAG GGT CGT GCT TTC TTC TTC AAA
GAC GTG ACT GAG GCT GTT GCG AAC AAT TTC TTC CCG GGA GCT ACA
GGT ACT AAT CGT ATC AAA TTA ACC TTT ACA GGT TCT TAT GGC GAT
CTC GAG AAA AAC GGC GGA CTA CGT AAG GAC AAT CCC CTA GGT ATC
TTC CGT CTG GAA AAC TCG ATA GTT AAC ATT TAT GGC AAA GCT GGT
GAC GTT AAA AAA CAG GCT AAA TTC TTC TTA CTG GCT ATC CAG ATG
GTT TCG GAG GCT GCG CGC TTT AAG TAT ATC AGT GAC AAA ATC CCG
TCT GAA AAA TAC GAA GAA GTT ACC GTT GAC GAA TAC ATG ACC GCT
CTG GAA AAC AAC TGG GCT AAA CTG TCT ACG GCC GTA TAC AAC TCT
AAG CCT TCT ACC ACC ACC GCT ACC AAA TCT CAA CTG GCT ACC TCT
CCG GTT ACC ATC TCT CCG TGG ATA TTC AAA ACC GTC GAG GAA ATC
AAA CTG GTT ATG GGT CTG CTT AAG TCT TCT TAA TAA ATC GAT

4. Rekombinantes Plasmid mit dem Gen nach Anspruch 3, wobei das Gen in das Plasmid integriert vorliegt.

5. Rekombinantes Plasmid mit dem Gen nach Anspruch 3, wobei das Gen in den MAP-Genabschnitt des Plasmids pSH7 insertiert vorliegt, wobei das Gen derart integriert ist, daß das Gen in E.coli exprimiert und das produzierte Protein sezerniert wird.

6. E.coli, transformiert mit dem Plasmid nach Anspruch 5.

7. Antivirales Protein, erhältlich durch Kultivieren der E.coli-Transformante nach Anspruch 6, die das Gen nach Anspruch 3 exprimiert, um das Protein zu produzieren, und Sezernieren des Proteins in ein Medium.

## Revendications

1. Protéine antivirale ayant la séquence d'acides aminés suivants :

Ala Pro Thr Leu Glu Thr Ile Ala Ser Leu Asp Leu Asn Asn Pro
1               5                   10                  15
Thr Thr Tyr Leu Ser Phe Ile Thr Asn Ile Arg Thr Lys Val Ala
                20                  25                  30
Asp Lys Thr Glu Gln Ser Thr Ile Gln Lys Ile Ser Lys Thr Phe
                35                  40                  45
Thr Gln Arg Tyr Ser Tyr Ile Asp Leu Ile Val Ser Ser Thr Gln
                50                  55                  60
Lys Ile Thr Leu Ala Ile Asp Met Ala Asp Leu Tyr Val Leu Gly
                65                  70                  75
Tyr Ser Asp Ile Ala Asn Asn Lys Gly Arg Ala Phe Phe Phe Lys
                80                  85                  90
Asp Val Thr Glu Ala Val Ala Asn Asn Phe Phe Pro Gly Ala Thr
                95                  100                 105
Gly Thr Asn Arg Ile Lys Leu Thr Phe Thr Gly Ser Tyr Gly Asp
                110                 115                 120
Leu Glu Lys Asn Gly Gly Leu Arg Lys Asp Asn Pro Leu Gly Ile
                125                 130                 135
Phe Arg Leu Glu Asn Ser Ile Val Asn Ile Tyr Gly Lys Ala Gly
                140                 145                 150
Asp Val Lys Lys Gln Ala Lys Phe Phe Leu Leu Ala Ile Gln Met
                155                 160                 165
Val Ser Glu Ala Ala Arg Phe Lys Tyr Ile Ser Asp Lys Ile Pro
                170                 175                 180
Ser Glu Lys Tyr Glu Glu Val Thr Val Asp Glu Tyr Met Thr Ala
                185                 190                 195
Leu Glu Asn Asn Trp Ala Lys Leu Ser Thr Ala Val Tyr Asn Ser
                200                 205                 210

Lys Pro Ser Thr Thr Thr Ala Thr Lys Ser Gln Leu Ala Thr Ser
                215                 220                 225
Pro Val Thr Ile Ser Pro Trp Ile Phe Lys Thr Val Glu Glu Ile
                230                 235                 240
Lys Leu Val Met Gly Leu Leu Lys Ser Ser
                245                 250

2. Gène codant la protéine telle que définie dans la revendication 1.

3. Gène selon la revendication 2 qui a la séquence de bases suivante :

GCG CCT ACT CTA GAA ACC ATC GCT TCT CTG GAC CTG AAC AAC CCG
ACC ACC TAC CTG TCT TTC ATA ACG AAT ATC CGT ACG AAA GTC GCA
GAC AAA ACC GAA CAG TCG ACC ATC CAG AAA ATC TCT AAA ACC TTC
ACC CAG CGT TAC TCT TAC ATA GAC TTG ATC GTG AGC TCG ACG CAG
AAA ATC ACC CTA GCT ATC GAC ATG GCT GAC CTG TAC GTT CTG GGT
TAC TCT GAC ATC GCT AAT AAC AAG GGT CGT GCT TTC TTC TTC AAA
GAC GTG ACT GAG GCT GTT GCG AAC AAT TTC TTC CCG GGA GCT ACA
GGT ACT AAT CGT ATC AAA TTA ACC TTT ACA GGT TCT TAT GGC GAT
CTC GAG AAA AAC GGC GGA CTA CGT AAG GAC AAT CCC CTA GGT ATC
TTC CGT CTG GAA AAC TCG ATA GTT AAC ATT TAT GGC AAA GCT GGT
GAC GTT AAA AAA CAG GCT AAA TTC TTC TTA CTG GCT ATC CAG ATG
GTT TCG GAG GCT GCG CGC TTT AAG TAT ATC AGT GAC AAA ATC CCG
TCT GAA AAA TAC GAA GAA GTT ACC GTT GAC GAA TAC ATG ACC GCT
CTG GAA AAC AAC TGG GCT AAA CTG TCT ACG GCC GTA TAC AAC TCT
AAG CCT TCT ACC ACC ACC GCT ACC AAA TCT CAA CTG GCT ACC TCT
CCG GTT ACC ATC TCT CCG TGG ATA TTC AAA ACC GTC GAG GAA ATC
AAA CTG GTT ATG GGT CTG CTT AAG TCT TCT TAA TAA ATC GAT

4. Plasmide recombiné comprenant le gène tel que défini dans la revendication 3, ledit gène étant intégré dans ledit plasmide.

5. Plasmide recombiné comprenant le gène tel que défini dans la revendication 3, ledit gène étant inséré dans une région d'un gène de la PAM du plasmide pSH7, dans lequel ledit gène est intégré de sorte que ledit gène est exprimé dans E. coli et que la protéine produite est sécrétée.

6. E. coli transformée avec le plasmide tel que défini dans la revendication 5.

7. Protéine antivirale qui peut être obtenue par culture du transformant de E. coli tel que défini dans la revendication 6, expression du gène tel que défini dans la revendication 3 pour produire ladite protéine et sécrétion de ladite protéine dans un milieu.
